(19) 

**Europäisches Patentamt**
**European Patent Office**
**Office européen des brevets**

(11) **EP 2 520 222 A1**

(12) **EUROPEAN PATENT APPLICATION**

(43) Date of publication:
**07.11.2012 Bulletin 2012/45**

(51) Int Cl.:
**A61B 5/0205** (2006.01)    **A61B 5/024** (2006.01)
**A61B 5/11** (2006.01)

(21) Application number: **12166550.9**

(22) Date of filing: **03.05.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **06.05.2011  JP 2011103448**

(71) Applicant: **Seiko Epson Corporation**
**Tokyo 163-0811 (JP)**

(72) Inventor: **Kuroda, Masao**
**Nagano, 392-8502 (JP)**

(74) Representative: **HOFFMANN EITLE**
**Patent- und Rechtsanwälte**
**Arabellastrasse 4**
**81925 München (DE)**

(54) **Biological information processing device**

(57)    A biological information processing device, including: a measuring unit that measures the pulse rate of a test subject; a detector that detects a body motion of the test subject; a computation unit that computes an intensity of exercise performed by the test subject using a result of detection by the detector; a first estimation unit that estimates an estimated pulse rate using the exercise intensity computed by the computation unit; and a controller that controls so that the pulse rate from a result of the measurement is displayed in an instance in which measurement by the measuring unit is possible, and the estimated pulse rate estimated by the first estimation unit is displayed in an instance in which measurement by the measuring unit is not possible.

Fig. 4

EP 2 520 222 A1

**Description**

**CROSS-REFERENCE TO RELATED APPLICATIONS**

This application claims priority to Japanese Patent Application No. 2011-103448 filed on May 6, 2011. The entire disclosure of Japanese Patent Application No. 2011-103448 is hereby incorporated herein by reference.

**BACKGROUND**

**Technical Field**

The present invention relates to a biological information processing device for estimating the pulse rate.

**Background Technology**

There has been known, as biological information processing devices used by a test subject to manage exercise and health, pulse rate monitors that are worn on a part of the test subject's body and used to measure the pulse rate of the test subject. A pulse rate monitor is used to detect variations in the blood flow volume in the test subject wearing the device and to measure the pulse rate of the test subject. Known pulse rate monitors include those that use light, those that use ultrasound, or those that use infrared light.

In order to correctly measure the pulse rate, it is necessary to detect the variation in the blood flow volume in the test subject with precision. One factor that causes a fall in the accuracy of detection is the body motion of the test subject. This is because body motion disrupts the blood flow in the test subject. In particular, small movements such as closing or opening of hands are difficult to detect and make it more difficult to specify the pulse rate. In Patent Citation 1, there is disclosed a technique for using a pressure sensor or a load sensor to detect a small movement by the test subject and taking the small movement into consideration when measuring the pulse rate.

Another factor that causes a fall in the accuracy of measuring the pulse rate is a fall in the external air temperature. A fall in the external air temperature reduces the blood flow volume and makes it more difficult to detect the pulse wave signal itself. In Patent Citation 2, there is disclosed a technique for taking variation in the external air temperature into consideration when measuring the pulse rate.

Japanese Laid-open Patent Publication No. 2004-283228 (Patent Document 1) and Japanese Laid-open Patent Publication No. 2009-34366 (Patent Document 2) are examples of the related art.

There are a variety of factors that cause a fall in the accuracy of pulse rate measurement. Other than the body motion or the external air temperature described above, the accuracy of pulse rate measurement is also influenced by the constitution of the test subject, the type and amount of clothing worn, and other factors. The intensity of a detected pulse wave signal may, in an extreme instance, fall to about 1/20 of the intensity in an ideal state.

Also, in a scenario in which the test subject wears a pulse rate monitor at all times in everyday life, there are instances in which it becomes difficult to measure the pulse rate due to a variation in the position at which the pulse rate monitor is being worn. In other words, there is a possibility of a situation in which the test subject performing a movement causes the position at which the pulse rate monitor is being worn to become displaced from an ideal wearing position, making it difficult for the pulse rate to be measured in an accurate manner.

**SUMMARY**

With the above-mentioned problems in view, an advantage of the present invention is to propose a new method for correctly estimating the pulse rate.

A first aspect for solving the above-mentioned problems is a biological information processing device, including: a measuring unit that measures the pulse rate of a test subject; a detector that detects a body motion of the test subject; a computation unit that computes an intensity of exercise performed by the test subject using a result of detection by the detector; a first estimation unit that estimates an estimated pulse rate using the exercise intensity computed by the computation unit; and a that controls so that the pulse rate from a result of the measurement is displayed in an instance in which measurement by the measuring unit is possible, and the estimated pulse rate estimated by the first estimation unit is displayed in an instance in which measurement by the measuring unit is not possible.

According to the first aspect, the pulse rate measuring unit measures the pulse rate of the test subject and the detector detects the body motion of the test subject. The computation unit then uses the result of detection by the detector to compute the intensity of exercise performed by the test subject. Exercise intensity is the vigorousness of exercise performed by the test subject, and correlates with the pulse rate of the test subject. Therefore, the first estimation unit estimates the estimated pulse rate using the exercise intensity computed by the computation unit. Using exercise intensity makes it possible to estimate the pulse rate with a high degree of accuracy, even in an instance in which measurement of the pulse rate is difficult.

As a second aspect, the biological information processing device according to the first aspect may be configured so that the estimation unit has a second estimation unit that obtains a provisional pulse rate using the exercise intensity computed by the computation unit; and obtains the estimated pulse rate so as to progressively approach, in accordance with the passage of time, the provisional pulse rate from a last pulse rate to be measured by the measuring unit.

When the test subject performs an exercise, the pulse rate changes in a transient manner. Therefore, as in the second aspect, the first estimation unit uses the exercise intensity computed by the computation unit to obtain a provisional pulse rate. Therefore, the estimated pulse rate is obtained so as to progressively approach, in accordance with the passage of time, the provisional pulse rate from the last pulse rate to be measured by the measuring unit, whereby the pulse rate can be estimated so as to reflect the actual time change in the pulse rate.

More specifically, as a third aspect, the biological information processing device according to the second aspect may be configured so that the second estimation unit obtains, in an instance in which the last pulse rate to be measured by the measuring unit is lower than the provisional pulse rate, obtains the estimated pulse rate so that the pulse rate changes logarithmically in accordance with the passage of time.

In a situation in which the pulse rate is increasing, such as at the start of exercise, the pulse rate tends to rise relatively quickly and then gradually stabilize. Therefore, in an instance in which the last pulse rate to be measured by the measuring unit is lower than the provisional pulse rate as in the third aspect, the second estimation unit obtains the estimated pulse rate so that the pulse rate changes logarithmically in accordance with the passage of time, whereby the pulse rate can be estimated so as to follow the actual time change in the pulse rate.

As a fourth aspect, the biological information processing device according to the second or third aspects can be configured so that the second estimation unit obtains, in an instance in which the last pulse rate to be obtained by the measuring unit is higher than the provisional pulse rate, obtains the estimated pulse rate so that the pulse rate changes sigmoidally in accordance with the passage of time.

In a situation in which the pulse rate is falling, such as at the end of exercise, the pulse rate tends to initially drop relatively insignificantly, rapidly fall after a certain time has elapsed, and then slowly converge. Therefore, in an instance in which the last pulse rate to be measured by the measuring unit is higher than the provisional pulse rate as in the fourth aspect, the second estimation unit obtains the estimated pulse rate so that the pulse rate changes sigmoidally in accordance with the passage of time, whereby the pulse rate can be estimated so as to follow the actual time change in the pulse rate.

As a fifth aspect, the biological information processing device according to any of the second through fourth aspects may be configured so that in the second estimation unit, a minimum value of the provisional pulse rate is a lowest pulse rate measured by the measuring unit.

According to the fifth aspect, in the second estimation unit, the minimum value of the provisional pulse rate is the lowest pulse rate measured by the measuring unit. It is thereby possible to estimate the pulse rate so that even if the provisional pulse rate falls below the lowest pulse rate measured by the measuring unit, the estimated pulse rate does not fall below this lowest pulse rate. Also, while the converging pulse rate differs between different individuals, using the lowest pulse rate measured by the measuring unit as the minimum value of the provisional pulse rate makes it possible to take the individual differences into account.

As a sixth aspect, the biological information processing device according to the first through fifth aspects may be configured so that the detector is configured so as to have at least one of an acceleration sensor and a velocity sensor.

According to the sixth aspect, the body motion detector is configured so as to have at least one of an acceleration sensor and a velocity sensor. These sensors make it possible for the acceleration or velocity of the test subject to be detected as body motion using a simple configuration.

As a seventh aspect, the biological information processing device according to any of the first through sixth aspects can be configured so that the detector is configured so as to have at least an acceleration sensor, and the computation unit calculates a pitch of the test subject from an acceleration detected by the acceleration sensor, and thereby computes the intensity of exercise performed by the test subject.

According to the seventh aspect, the detector is configured so as to have at least an acceleration sensor. The acceleration sensor makes it possible for the acceleration of the test subject as body motion using a simple configuration. By then calculating the pitch of the test subject using the detected acceleration, the intensity of exercise performed by the test subject can be obtained in an appropriate manner. There may be an instance in which the acceleration sensor is provided to equipment for the purpose of removing the body motion component from the pulse wave signal. In such an instance, the acceleration sensor can be used for two purposes: for removing the body motion component and for estimating the exercise intensity.

As an eighth aspect, the biological information processing device according to the first through seventh aspects may further comprise a judging unit that judges, on the basis of the exercise intensity computed by the computation unit and the pulse rate measured by the measuring unit, whether or not the test subject is in a stable state; wherein the first estimation unit has a third estimation unit in which the pulse rate corresponding to an instance in which the test subject is judged to be in the stable state is used as the estimated pulse rate within a period where, after the judging unit judges that the test subject in the stable state, a measurement cannot be performed by the measuring unit, and the exercise intensity corresponding to an instance in which the test subject has been judged to be in the stable state and the exercise intensity computed by the computation unit satisfy a predetermined uniform-intensity condition.

According to the eighth aspect, the judging unit can judge, in a simple and appropriate manner, whether or not the test subject is in a stable state, by basing the judging on the exercise intensity computed by the computation unit and the pulse rate measured by the measuring unit. It is thought that no large change will occur in the pulse rate if the test subject is in a stable state. Therefore, the pulse rate corresponding to an instance in which the test subject is judged to be in the stable state is used as the estimated pulse rate within a period where, after the judging unit judges that the test subject in the stable state, a measurement cannot be performed by the measuring unit and the exercise intensity corresponding to an instance in which the test subject has been judged to be in the stable state and the exercise intensity computed by the computation unit satisfy a predetermined uniform-intensity condition. It is thereby possible to estimate the pulse rate in a suitable manner, taking the state of the test subject into account.

## BRIEF DESCRIPTION OF THE DRAWINGS

Referring now to the attached drawings which form a part of this original disclosure:

FIG. 1 is a front view of a pulse rate monitor;

FIG. 2A is a reverse-surface view of the pulse rate monitor;

FIG. 2B shows the pulse rate monitor in a state of use;

FIG. 3 illustrates the action of the pulse wave sensor;

FIG. 4 is a block diagram showing a functional configuration of the pulse rate monitor;

FIG. 5 is a graph showing the time change in the pulse rate and the pitch of the test subject before, during, and after exercise;

FIG. 6A shows a start-of-exercise model;

FIG. 6B shows an end-of-exercise model;

FIG. 7 is a flow chart showing the flow of a main process;

FIG. 8 is a flow chart showing the flow of a pitch-stability-judging process;

FIG. 9 is a flow chart showing the flow of the pulse-stability-judging process; and

FIG. 10 is a flow chart showing the pulse rate estimation process.

## DETAILED DESCRIPTION OF EXEMPLARY EMBODIMENTS

A preferred embodiment of the present invention will now be described with reference to the accompanying drawings. The present embodiment is an embodiment in which the biological information processing device according to the present invention is applied to a wristwatch-type pulse rate monitor. It shall be apparent that configurations in which the present invention can be applied are not limited to the embodiment described below.

## 1. External appearance configuration

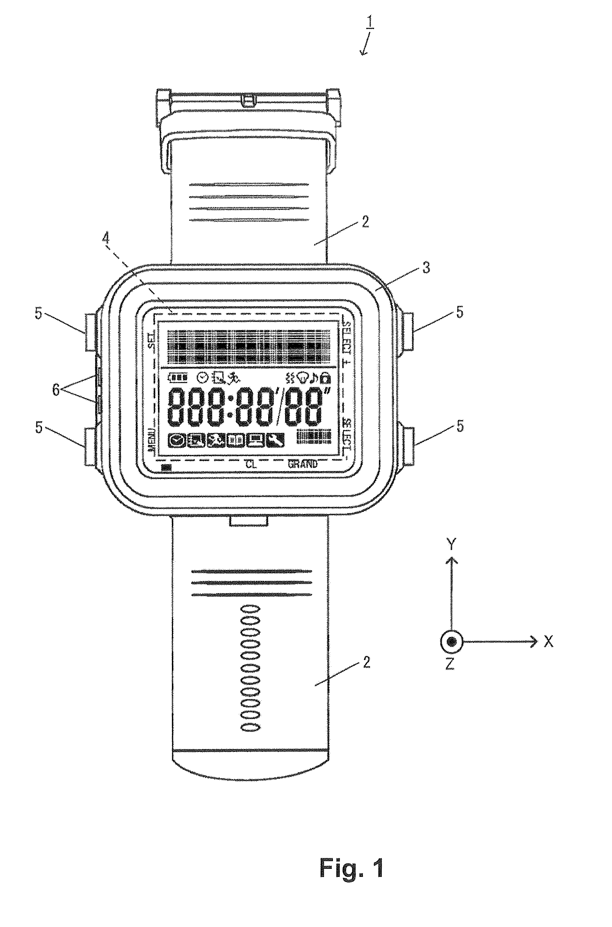

FIG. 1 is a front view of a pulse rate monitor 1 according to the present embodiment. The pulse rate monitor 1 is provided with a wristband 2. A liquid crystal display 4 for displaying the time, an operation state of the pulse rate monitor 1, and a variety of biological information (e.g., pulse rate, exercise intensity, calorie consumption) using, e.g., text, numerals, or icons, is arranged on a case 3.

Operation buttons 5 used to operate the pulse rate monitor 1 are provided to a periphery section (side surface) of the case 3. The pulse rate monitor 1 operates using, e.g., an internally provided secondary cell as a power source. A charge terminal 6, used to connect with an external charger and charge the internally provided secondary cell, is provided on the side surface of the case 3.

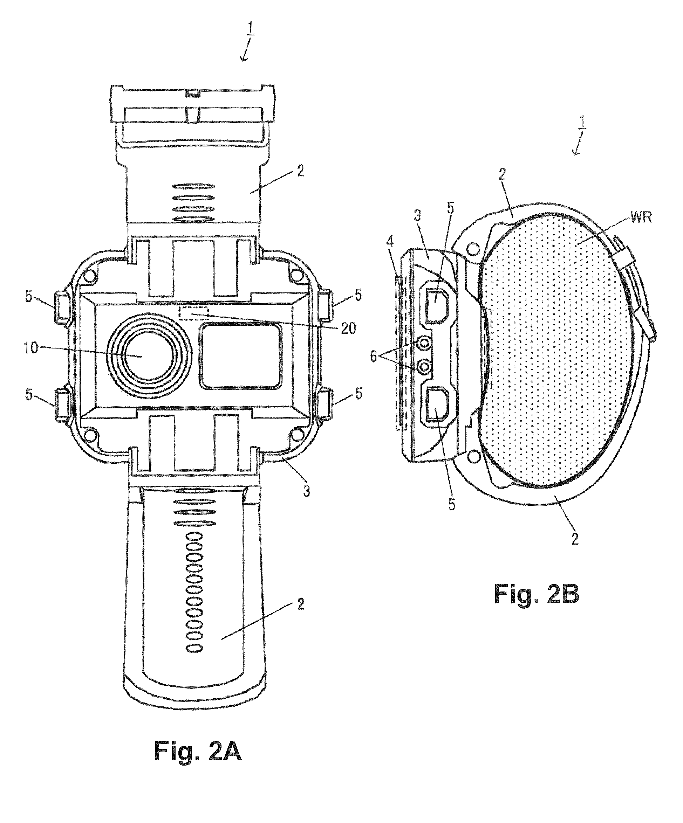

FIG. 2A is a reverse surface view of the pulse rate monitor 1, and shows an external view of the pulse rate monitor 1 when viewed from a reverse surface of the case 3. FIG. 2B shows the pulse rate monitor 1 in a state of use, and shows a side view of the pulse rate monitor 1 when in a state of being worn on the wrist WR of the test subject.

A pulse wave sensor 10 for detecting a pulse wave of the test subject and outputting a pulse wave signal is provided on the reverse surface of the case 3. The pulse wave sensor 10 detects the pulse wave at the wrist WR, which is in contact with the reverse surface of the case 3. In the present embodiment, the pulse wave sensor 10 is a photoelectric pulse wave sensor, and includes a mechanism for optical detection of the pulse wave.

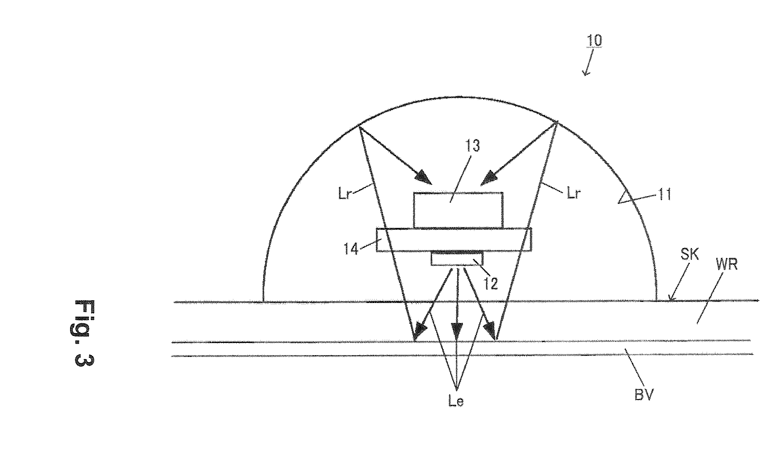

FIG. 3 is an expanded view of an internal structure of the pulse wave sensor 10 when viewed from a side surface of the case 3. The pulse wave sensor 10 is installed in a hemispherical accommodating space having a circular bottom surface, formed on the reverse-surface-side of the case 3. A light-emitting diode (LED) or another light source 12 and a phototransistor or another light-receiving element 13 are internally provided within the accommodating space. An internal surface of the hemisphere is a mirror surface 11. When the bottom-surface-side of the hemisphere is defined as the downward direction, the light-receiving element 13 and the light source 12 are respectively installed on an upper surface and a lower surface of a substrate 14.

When the light source 12 emits light Le towards the skin SK of the wrist WR of the user, the emitted light Le is reflected by a blood vessel BV under the skin, and returns to within the hemisphere as reflected light Lr. The reflected light Lr is further reflected by the hemispherical mirror surface 11, and strikes the light-receiving element 13 from above.

A light-absorbing action of hemoglobin in the blood causes the intensity of the reflected light Lr from the blood vessel BV to fluctuate so as to reflect the fluctuation in blood flow. The pulse wave sensor 10 causes the light source 12 to blink at a predetermined period that is faster than that of pulsation. Each time the light source 12 illuminates, the light-receiving element 13 outputs, through photoelectric conversion, a pulse wave signal that corresponds to the intensity of received light. The pulse wave sensor 10 causes the light source 12 to blink at a frequency of, e.g., 128 Hz.

As shown in FIG. 2A, the pulse rate monitor 1 is internally provided with a body motion sensor 20 for detecting the body motion of the test subject. In the present embodiment, the body motion sensor 20 is configured so as to have at least an acceleration sensor. The acceleration sensor is an acceleration sensor for three axes, namely, e.g., a z-axis oriented along a direction normal to a cover glass surface of the case 3 so that a display-surface-side is positive; a y-axis oriented along the up-down direction so that a direction oriented at 12 o'clock on a clock is positive; and an x-axis oriented along the left-right direction so that a direction oriented at 3 o'clock on the clock is positive, as shown in FIG. 1. In a state in which the pulse rate monitor 1 is being worn, the x-axis coincides with a direction oriented from the elbow to the wrist of the test subject. The body motion sensor 20 detects acceleration along each of the three axes x, y, and z, and outputs a result as a body motion signal.

## 2. Principle

The pulse rate monitor 1 measures the pulse rate on the basis of a pulse wave signal detected by the pulse wave sensor 10. The pulse wave signal is a signal in which a pulsation component signal and a body motion noise component signal for the test subject are superimposed on each other. Therefore, on the basis of the body motion signal outputted from the body motion sensor 20, the pulse rate monitor 1 removes the body motion noise component signal, and extracts the pulsation component signal, from the pulse wave signal. The pulse rate monitor 1 then measures the pulse rate on the basis of the extracted pulsation component signal.

Specifically, e.g., a finite impulse response (FIR) filter or another digital filter is configured as an adaptive filter, and the adaptive filter is used to execute a process for removing the body motion noise component from the pulse wave signal as a digital signal process. Then, frequency analysis is performed on the extracted pulsation component signal to specify a pulsation-indicating spectrum, and the pulse rate is measured based on the corresponding frequency (or period). For example, a fast Fourier transform (FFT) can be applied for the frequency analysis.

In the present embodiment, assuming a situation in which measurement of the pulse rate is difficult in an instance in which the above-mentioned method is used, a result of detection, by the body motion sensor 20, of the body motion of the test subject is used to compute the intensity of exercise performed by the test subject. The computed exercise intensity is used to estimate the pulse rate of the test subject.

## 2-1 Estimating the pulse rate using exercise intensity

In the present embodiment, an acceleration sensor is used to detect the acceleration of the test subject as body motion. The pitch (walking pace) of the test subject is calculated from the acceleration detected by the acceleration sensor, whereby the intensity of exercise performed by the test subject is computed. The computation of the pitch can be performed by performing a predetermined frequency analysis (e.g., FFT) on an acceleration signal outputted from the acceleration sensor and specifying and extracting a frequency component corresponding to the pitch. Details of the computation method are disclosed in, e.g., JP-A 2004-81745.

Next, the pulse rate is estimated from the pitch on the basis of a predefined correspondence relationship between pitch and pulse rate. In the present embodiment, the pulse rate is estimated from the pitch according to an approximation formula shown in the following Equation (1).

[Equation 1]

$$HR(p) = A \cdot p^2 + B \cdot p + C \cdots (1)$$

Here, p represents pitch, and HR(p) represents the pulse rate estimated from the pitch *p*. A, B, and C are the quadratic coefficient, the linear coefficient, and the constant term of the approximation formula, respectively.

There is a reason why a quadratic function is used to define the correspondence relationship between the pitch and the pulse rate as shown in Equation 1. Exercise intensity can be broadly divided into two types: physiological exercise intensity and physical exercise intensity. Physiological exercise intensity is exercise intensity measured by physiological information about the test subject, and includes representations of, e.g., oxygen uptake or pulse rate. Physical exercise intensity is exercise intensity measured by physical information about the test subject, and includes representations of, e.g., speed or time. Pitch, used as exercise intensity in the present embodiment, is a type of physical exercise intensity.

From amongst types of physiological exercise intensity, the relative pulse rate %HRR is widely used as a representation of the pulse rate. The relative pulse rate %HRR is given by the following Equation (2).

[Equation 2]

$$\%HRR = \frac{HR - HR_{rest}}{HR_{max} - HR_{rest}} \cdots (2)$$

HR represents an unknown pulse rate of the test subject. $HR_{max}$ and $HR_{rest}$ are a known maximum pulse rate and a known rest pulse rate of the test subject, respectively.

From amongst types of physiological exercise intensity, relative oxygen uptake %VO$_2$R is widely used as a representation of oxygen uptake. The relative oxygen uptake %VO$_2$R is given by the following Equation (3).

[Equation 3]

$$\%VO_2R = \frac{VO_2 - VO_{2rest}}{VO_{2max} - VO_{2rest}} \cdots (3)$$

VO$_2$ represents an unknown oxygen uptake of the test subject. VO$_{2max}$ and VO$_{2rest}$ are a known maximum oxygen uptake and a known rest oxygen uptake of the test subject, respectively.

The oxygen uptake VO$_2$ of the test subject in Equation (3) can be estimated using the travel speed v of the test subject. Specifically, the oxygen uptake VO$_2$ can be approximated, e.g., according to the following Equation (4).

[Equation 4]

$$VO_2 = 0.1 \cdot v + VO_{2rest} \cdots (4)$$

Equation (4) was formulated after an estimation method proposed by the American College of Sports Medicine.

A correlation is known to exist between the travel speed v of the test subject and the pitch p of the test subject. For example, it is known that the travel speed v can be approximated according to the following Equation (5).

[Equation 5]

$$v = (0.001 \cdot p + 0.37 \cdot h) \cdot p \cdots (5)$$

Here, h represents the height of the test subject.

Equations (2) through (5) link the pitch *p* of the test subject and the relative oxygen uptake %VO$_2$R. In other words, by computing the pitch *p* of the test subject as a measure of physical exercise intensity, it is possible to compute the relative oxygen uptake %VO$_2$R as a measure of physiological exercise intensity. The inventor of the present invention postulated that the relative oxygen uptake %VO$_2$R calculated from the physical exercise intensity coincides with the relative pulse rate %HRR as a measure of physiological exercise intensity. Specifically, the following Equation (6) was

postulated.

[Equation 6]

$$\%HRR = \%VO_2R \cdots (6)$$

It can be seen from Equations (2) through (5) that the relative oxygen uptake $\%VO_2R$ is represented by a quadratic function of the pitch p. Therefore, using the postulation of Equation (6), the relative pulse rate %HRR is also represented by a quadratic function of the pitch p. Using Equation (2), since the relative pulse rate %HRR is in a linear relationship with the pulse rate HR, the pulse rate HR can also be approximated by a quadratic function of the pitch $p$ as in Equation (1).

The value of each of the coefficients A though C in Equation (1) can be determined by, e.g., collecting, as sample data, data representing the pitch p and the pulse rate HR for a large number of test subjects, and fitting the sample data onto a quadratic curve. It is also possible to collect and fit sample data representing the pitch p and the pulse rate HR in relation to each of the test subjects, and to determine the value of each of the coefficients A through C for every test subject.

**2-2 Estimating the pulse rate in transient state**

In addition to estimating the pulse rate from the pitch as described above, in the present embodiment, the pulse rate in a transient state is also estimated. A transient state refers to a state at an intermediate stage of transition between one state and another state. In the present embodiment, a situation in which a test subject wearing the pulse rate monitor 1 is performing an exercise is assumed, and two transition states, namely (1) "start of exercise → during exercise", and (2) "during exercise → end of exercise", are considered transient states.

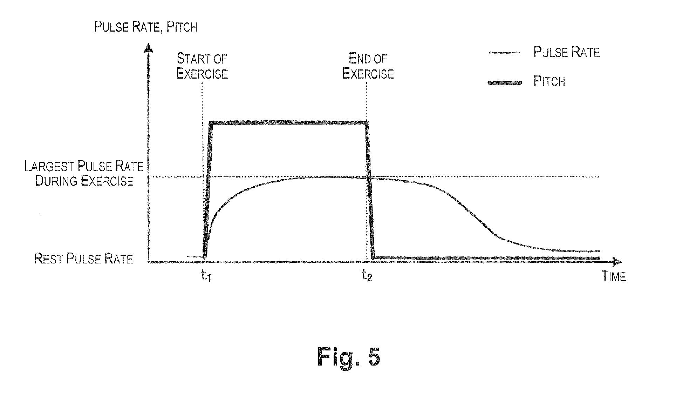

FIG. 5 is a graph showing the time variation in the pulse rate and the pitch of the test subject before, during, and after exercise; and is a graph created from data obtained through actual measurement. In FIG. 5, the horizontal axis represents the time axis, and the vertical axis represents the pulse rate and the pitch. The test subject starts exercising at time t1 and finishes exercising at time t2. This graph is one in which it is assumed that the exercise is of moderate load (e.g., walking).

**(1) Start of exercise → during exercise**

When the test subject starts exercising at time t1, the pitch rises rapidly. The pulse rate increases relatively fast with the rise in pitch, and undergoes a variation so as to converge to a maximum pulse rate during exercise. This time variation in the pulse rate is a logarithmic time variation.

**(2) During exercise → end of exercise**

When the test subject finishes exercise at time t2, the pitch falls rapidly. The pulse rate falls gradually with the fall in the pitch. Specifically, the pulse rate does not drop immediately when the exercise is ended, but starts to fall rapidly after a certain time has elapsed. The speed of the fall in the pulse rate subsequently diminishes again, and the pulse rate ultimately converges to a value that is slightly higher than the rest pulse rate. This time variation in the pulse rate is a sigmoid-shaped (S-shaped) time variation.

Thus, it can be seen that the disposition of the time variation in the pulse rate differs between the state corresponding to (1) "start of exercise → during exercise", and the state corresponding to (2) "during exercise → end of exercise". Therefore, two types of models are individually defined, namely a pulse rate estimation model in which the state corresponding to (1) "start of exercise → during exercise" is assumed (hereafter referred to as "start-of-exercise model") and a pulse rate estimation model in which the state corresponding to (2) "during exercise → end of exercise" is assumed (hereafter referred to as "end-of-exercise model"). A model corresponding to the state is selected and the pulse rate is estimated.

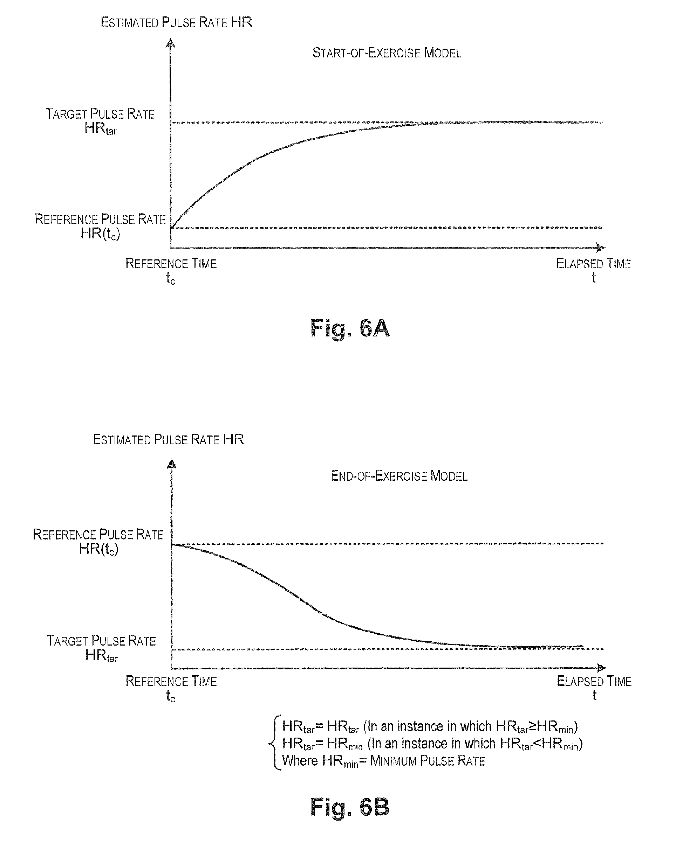

FIG. 6 illustrates the pulse rate estimation models. The start-of-exercise model is shown in FIG. 6A and the end-of-exercise model is shown in FIG. 6B. In the present embodiment, the pulse rate estimation models were defined as a function of elapsed time t after a given reference time tc.

In the present embodiment, the last time at which the pulse rate was measured in a state in which measurement of the pulse rate is possible on the basis of the pulse wave signal is set as the reference time tc. For convenience, the pulse rate at the reference time tc is defined as a reference pulse rate HR(tc). In other words, the reference pulse rate HR(tc) is the last pulse rate to be measured in a state in which measurement of the pulse rate is possible.

A pulse rate HR(p) estimated from the pitch p according to Equation (1) is set as a target pulse rate $HR_{tar}$. The target pulse rate $HR_{tar}$ is a pulse rate of the test subject obtained from the pitch p in a provisional manner (i.e., provisional pulse rate). An estimated pulse rate is obtained so as to progressively approach the target pulse rate $HR_{tar}$ from the reference pulse rate HR(tc) in accordance with the elapsed time t from the reference time tc. In other words, the exercise intensity is used to obtain a provisional pulse rate, and the estimated pulse rate is obtained so as to progressively approach, in accordance with the passage of time, the provisional pulse rate from the last pulse rate to be measured.

The start-of-exercise model is rendered in the form of a formula as a function in which with the progression of elapsed time t, the pulse rate increases logarithmically and progressively approaches the target pulse rate $HR_{tar}$. For example, using the start-of-exercise model rendered in the form of a formula as shown in the following Equation (7), an estimated pulse rate $HR_{up}(t)$ is obtained.

[Equation 7]

$$HR_{up}(t) = HR(t_c) + (HR_{tar} - HR(t_c)) \cdot \left\{ 1 - \exp\left( -\frac{\ln 2}{t_{up}} t \right) \right\} \cdots (7)$$

Here, $t_{up}$ represents a time width that determines the degree of increase (rate of increase) of the pulse rate.

The end-of-exercise model is rendered in the form of a formula as a function in which with the progression of elapsed time t, the pulse rate falls sigmoidally and progressively approaches the target pulse rate $HR_{tar}$. For example, using the end-of-exercise model rendered in the form of a formula as shown in the following Equation (8), an estimated pulse rate $HR_{down}(t)$ is obtained.

[Equation 8]

$$HR_{down}(t) = \frac{2(HR(t_c) - HR_{tar})}{\exp\left( \frac{\ln 2}{t_{down}} t \right) + 1} + HR_{tar} \cdots (8)$$

Here, $t_{down}$ represents the time width that determines the degree of decrease (rate of decrease) of the pulse rate.

A model that corresponds to the state corresponding to (1) "start of exercise → during exercise" or the state corresponding to (2) "during exercise → end of exercise" is selected, and the pulse rate is estimated. For example, in an instance in which the reference pulse rate HR(tc) is lower than the target pulse rate $HR_{tar}$, it can be thought that a state is present in which the pulse rate is increasing. Therefore, it is determined that the state corresponding to (1) "start of exercise → during exercise" is present, the start-of-exercise model is selected, and the pulse rate is estimated. In other words, in an instance in which the last pulse rate to be measured is lower than the provisional pulse rate, the estimated pulse rate is obtained so that the pulse rate changes logarithmically in accordance with the passage of time.

In contrast, in an instance in which the reference pulse rate HR(tc) is higher than the target pulse rate $HR_{tar}$, it is thought that a state is present in which the pulse rate is decreasing. Therefore, it is determined that the state corresponding to (2) "during exercise → end of exercise" is present, the end-of-exercise model is selected, and the pulse rate is estimated. In other words, in an instance in which the last pulse rate to be measured is higher than the provisional pulse rate, the estimated pulse rate is obtained so that the pulse rate changes sigmoidally in accordance with the passage of time.

Also, in the present embodiment, a minimum pulse rate $HR_{min}$ is set as a minimum value of the target pulse rate $HR_{tar}$, which is the provisional pulse rate. Normally, in a rest state before and after exercise, the pulse rate of the test subject often does not drop as far as the rest pulse rate $HR_{rest}$. In other words, as shown in FIG.5, the pulse rate of the test subject tends to be at a value that is slightly higher than that of the rest pulse rate before the start of exercise, and to converge at a value that is slightly higher than that of the rest pulse rate after the end of exercise. Therefore, an adjustment is made so that the target pulse rate $HR_{tar}$ does not become lower than the minimum pulse rate $HR_{min}$.

In the present embodiment, the minimum pulse rate $HR_{min}$ is set to a lowest pulse rate from among the pulse rates measured on the basis of the pulse wave signal. In an instance in which the target pulse rate is equal to or greater than the minimum pulse rate ($HR_{tar} \geq HR_{min}$), the target pulse rate is kept unchanged ($HR_{tar} = HR_{tar}$). In contrast, in an instance in which the target pulse rate is less than the minimum pulse rate ($HR_{tar} < HR_{min}$), the target pulse rate is set to the minimum pulse rate ($HR_{tar} = HR_{min}$).

## 3. Function configuration

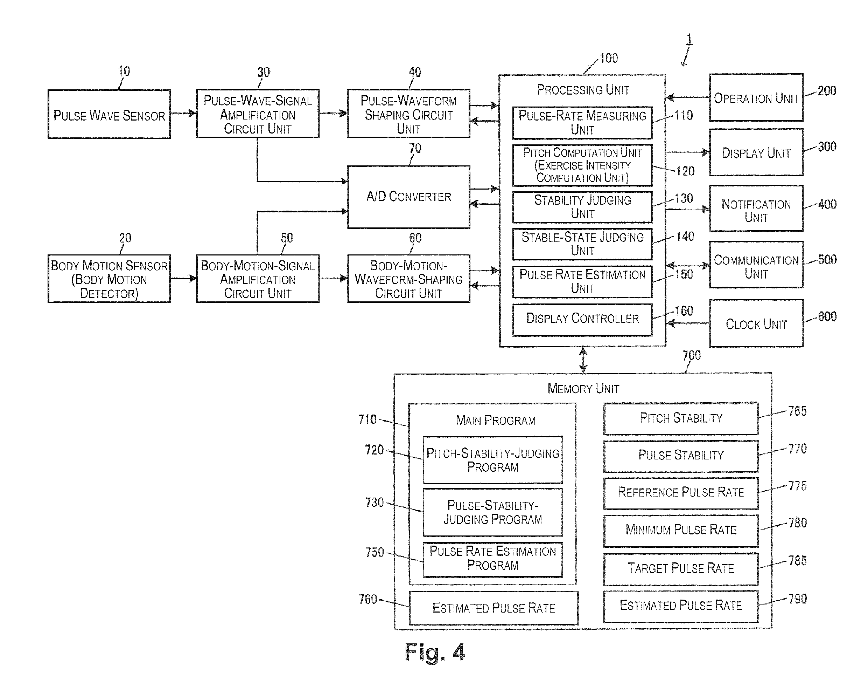

FIG. 4 is a block diagram showing an example of a functional configuration of the pulse rate monitor 1. The pulse rate monitor 1 is configured so as to include the pulse wave sensor 10, the body motion sensor 20, a pulse-wave-signal amplification circuit unit 30, a pulse-waveform shaping circuit unit 40, a body-motion-signal amplification circuit unit 50, a body-motion-waveform-shaping circuit unit 60, an analog-to-digital (A/D) converter 70, a processing unit 100, an operation unit 200, a display unit 300, a notification unit 400, a communication unit 500, a clock unit 600, and a memory unit 700.

The pulse wave sensor 10 is a sensor for measuring the pulse wave of a test subject wearing the pulse rate monitor 1, and is configured so as to have, e.g., a photoelectric pulse wave sensor. The pulse wave sensor 10 detects, as a pulse wave signal, a change in volume generated by inflow of blood into a body tissue, and outputs the pulse wave signal to the pulse-wave-signal amplification circuit unit 30.

The pulse-wave-signal amplification circuit unit 30 is an amplification circuit for amplifying, by a predetermined gain, the pulse wave signal inputted from the pulse wave sensor 10. The pulse-wave-signal amplification circuit unit 30 outputs the amplified pulse wave signal to the pulse-waveform shaping circuit unit 40 and the A/D converter 70.

The pulse-waveform shaping circuit unit 40 is a circuit unit for shaping the pulse wave signal that has been amplified by the pulse-wave-signal amplification circuit unit 30, and is configured so as to have a circuit for removing a high-frequency noise component, a clipping circuit, and other elements. The processing unit 100 judges, on the basis of a pulse waveform that has been shaped by the pulse-waveform shaping circuit unit 40, whether or not a pulse wave has been detected.

The body motion sensor 20 is a sensor for establishing the movement of the test subject wearing the pulse rate monitor 1, and is configured so as to have at least an acceleration sensor. The body motion sensor 20 corresponds to a body-motion-detector for detecting the body motion of the test subject.

The body-motion-signal amplification circuit unit 50 is an amplification circuit for amplifying, by a predetermined gain, the body motion signal inputted from the body motion sensor 20. The body-motion-signal amplification circuit unit 50 outputs the amplified body motion signal to the body-motion-waveform-shaping circuit unit 60 and the A/D converter 70.

The body-motion-waveform-shaping circuit unit 60 is a circuit unit for shaping the body motion signal that has been amplified by the body-motion-signal amplification circuit unit 50, and is configured so as to have a circuit for removing a high-frequency noise component, a circuit for differentiating between a gravitational acceleration component and other components, a clipping circuit, and other elements. The processing unit 100 judges, on the basis of a body motion waveform shaped by the body-motion-waveform-shaping circuit unit 60, whether or not body motion has been detected.

The A/D converter 70 samples and digitizes, at predetermined sampling time intervals, and converts to a digital signal, each of the analog pulse wave signal amplified by the pulse-wave-signal amplification circuit unit 30 and the analog body motion signal amplified by the body-motion-signal amplification circuit unit 50; the A/D converter 70 then outputs the converted digital signal to the processing unit 100.

The processing unit 100 is a control device and a computation device for performing overall control of each of the units of the pulse rate monitor 1 according to a variety of programs, such as a system program, stored in the memory unit 700, and is configured so as to have a processor such as a central processing unit (CPU). The processing unit 100 performs a main process according to a main program 710 stored in the memory unit 700, and performs a control so that the pulse rate of the test subject wearing the pulse rate monitor 1 is measured/estimated and displayed on the display unit 300.

The processing unit 100 has, as principal functional units, a pulse-rate measuring unit 110, a pitch computation unit 120, a stability judging unit 130, a stable-state judging unit 140, a pulse rate estimation unit 150, and a display controller 160.

The pulse-rate measuring unit 110 uses the body motion signal (body motion data) inputted from the A/D converter 70 to remove the body motion noise component from the pulse wave signal (pulse wave data), and uses the extracted pulsation component (pulsation data) to measure the pulse rate.

The pitch computation unit 120 uses the body motion signal (body motion data) inputted from the A/D converter 70 to compute the pitch of the test subject. The pitch computation unit 120 corresponds to an exercise intensity computation unit for computing the intensity of exercise performed by the test subject using a result of detection by the body-motion-detector.

The stability judging unit 130 judges the stability of the pitch and the pulse on the basis of the pitch computed by the pitch computation unit 120 and the pulse rate measured by the pulse-rate measuring unit 110. The stable-state judging unit 140 also judges whether or not the test subject is in a stable state on the basis of a result of stability judgement performed by the stability judging unit 130.

The pulse rate estimation unit 150 estimates the pulse rate according to the above-mentioned principle. The pulse rate estimation unit 150 corresponds to a pulse rate estimation unit for using the pitch p computed by the pitch computation unit 120 to estimate the estimated pulse rate.

The pulse rate estimation unit 150 has a gradual convergence estimation unit (not shown) for using the pitch to obtain a provisional pulse rate (target pulse rate), and for obtaining the estimated pulse rate so as to progressively approach, in accordance with the passage of time, the provisional pulse rate from the last pulse rate to be measured by the pulse-rate measuring unit 110 (reference pulse rate). The pulse rate estimation unit 150 also has a stable-state continuation estimation unit (not shown). The stable-state continuation estimation unit uses the pulse rate corresponding to an instance in which the test subject is judged to be in a stable state as the estimated pulse rate within a period where, after the stable-state judging unit 140 judges that the test subject in the stable state, a measurement cannot be performed by the pulse-rate measuring unit 110 and the exercise intensity (pitch) corresponding to an instance in which the test subject has been judged to be in the stable state and the exercise intensity (pitch) computed by the pitch computation unit 120 satisfy a predetermined uniform-intensity condition.

The display controller 160 performs a control so as to display a pulse rate measurement/estimation result on the display unit 300. In an instance in which measurement by the pulse-rate measuring unit 110 is possible, the display controller 160 causes the display unit 300 to display the pulse rate according to the measurement result. In contrast, in an instance in which measurement by the pulse-rate measuring unit 110 is not possible, the display unit 300 is caused to display the estimated pulse rate estimated by the pulse rate estimation unit 150.

The operation unit 200 is an input device configured so as to have a button switch or similar elements. The operation unit 200 outputs a signal corresponding to a pressed button to the processing unit 100. The operation unit 200 is operated to input a variety of commands, such as a command for measuring the pulse rate. The operation unit 200 corresponds to the operation buttons 5 shown in FIG. 1.

The display unit 300 is a display device configured so as to have a liquid crystal display (LCD) and other elements and used for performing a variety of displays on the basis of a display signal inputted from the processing unit 100. The display unit 300 displays a variety of biological information (pulse rate, exercise intensity, calorie consumption, etc.). The display unit 300 corresponds to the liquid crystal display 4 shown in FIG. 1.

The notification unit 400 is a notification device configured so as to have a speaker, a piezoelectric vibrator, or a similar device, and used for performing a variety of notifications based on a notification signal inputted from the processing unit 100. The notification unit 400 performs a variety of notifications for the benefit of the test subject by, e.g., outputting an alarm sound from a speaker or causing a piezoelectric vibrator to vibrate.

The communication unit 500 is a communication device for transmitting/receiving, in accordance with a control performed by the processing unit 100, information used within a device, with respect to a personal computer (PC) or another external information processing device. A variety of methods can be applied as a communication method for the communication unit 500, such as a format in which a wired connection is established through a cable that conforms to predetermined communication standards, a format in which a connection is established using an intermediate device known as a cradle that also functions as a charger, or a format in which a wireless connection is established using near field communication.

The clock unit 600 is a time-measuring device configured so as to have a device such as a crystal oscillator including a crystal unit and an oscillation circuit, and used for measuring the time of the pulse rate monitor 1. The time measured by the clock unit 600 is continually outputted to the processing unit 100.

The memory unit 700 is configured by read-only memory (ROM), flash ROM, random-access memory (RAM), or another memory device. The memory unit 700 stores a system program for the pulse rate monitor 1, and other programs, data, or other information for realizing a pulse rate measurement/estimation function, exercise intensity measurement function, calorie measurement function, and other functions. The memory unit 700 also has a work area for temporarily storing mid-process data, processing results, and other information relating to a variety of processes.

### 4. Data configuration

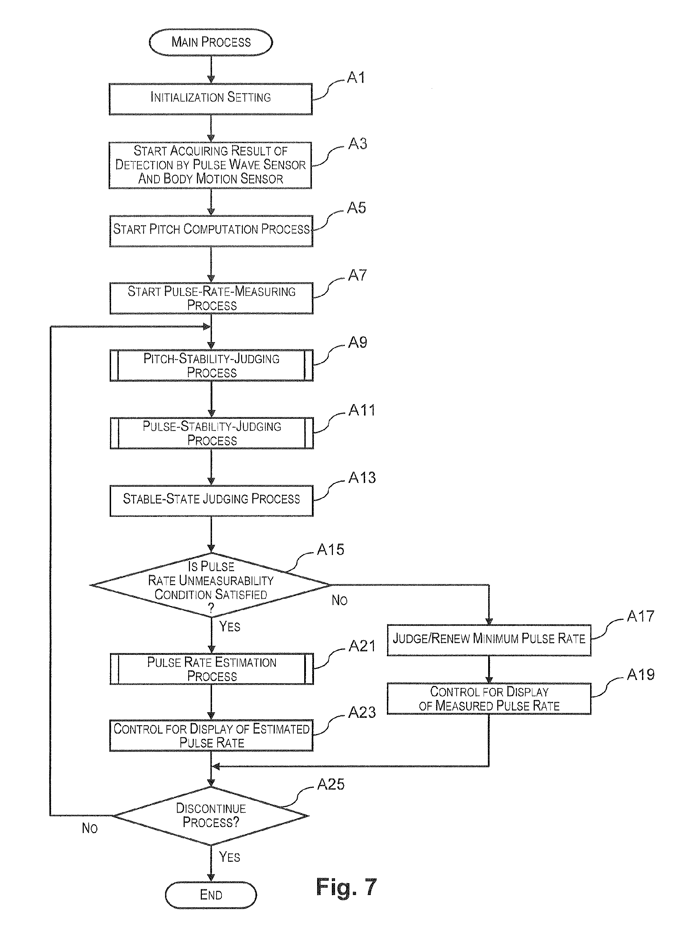

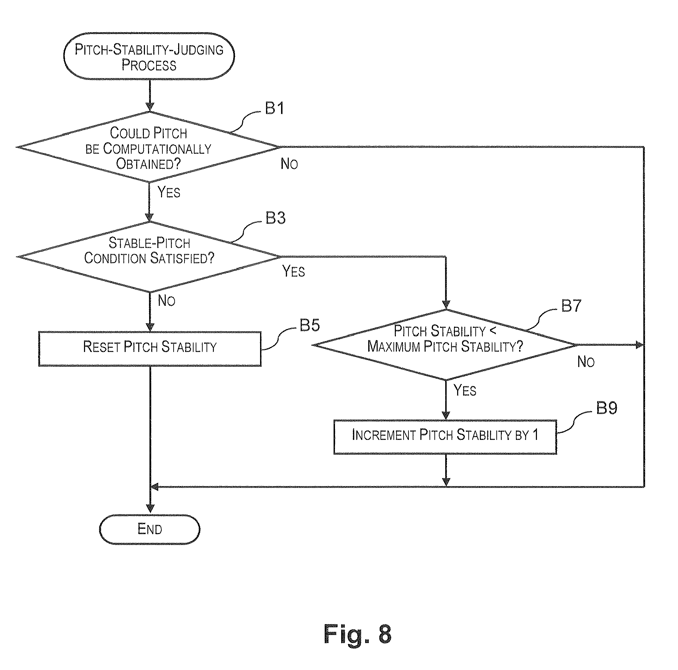

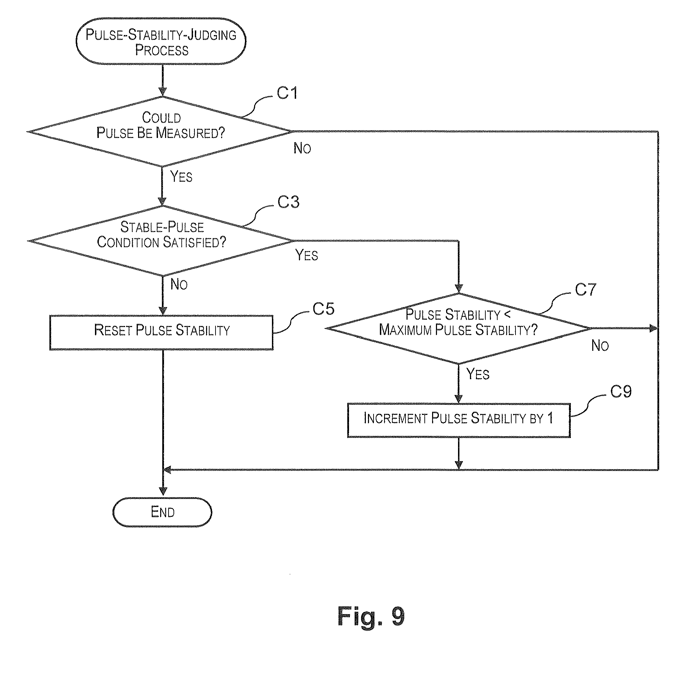

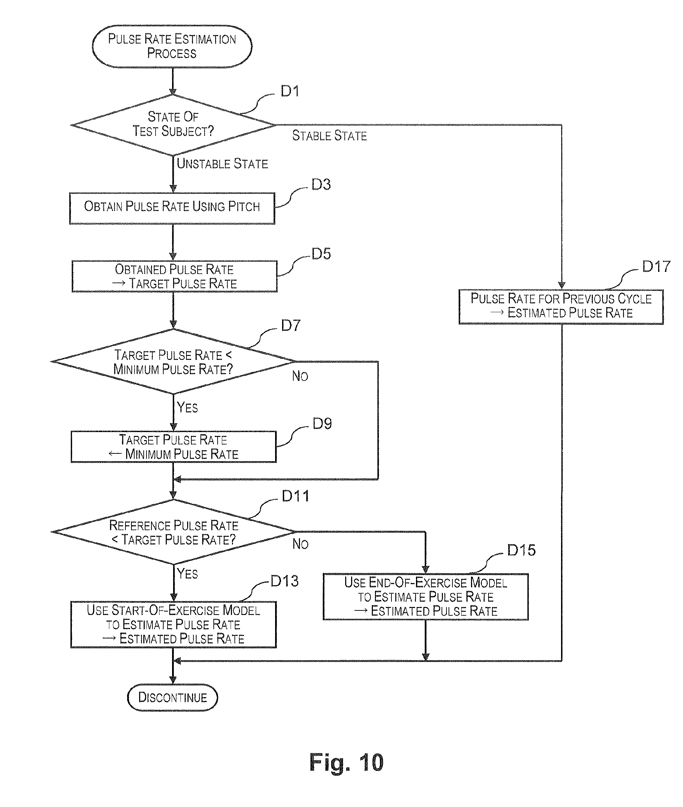

As shown in FIG. 4, the memory unit 700 stores the main program 710, which is executed as a main process (see FIG. 7). The main program 710 includes, as subroutines, a pitch-stability-judging program 720 executed as a pitch-stability-judging process (see FIG. 8), a pulse-stability-judging program 730 executed as a pulse-stability-judging process (see FIG. 9), and a pulse rate estimation program 750 executed as a pulse rate estimation process (see FIG. 10).

Also, the memory unit 700 stores, as data, e.g., an estimated pulse rate 760, a pitch stability 765, a pulse stability 770, a reference pulse rate 775, a minimum pulse rate 780, a target pulse rate 785, and an estimated pulse rate 790

### 5. Process flow

FIG. 7 is a flow chart showing the flow of a main process executed in the pulse rate monitor 1 due to the main program 710 stored in the memory unit 700 being read by the processing unit 100.

First, the processing unit 100 performs initialization setting (step A1). Specifically, the value of a variety of computation parameters used in the main process (e.g., the pitch stability 765 or the pulse stability 770) are initialized.

Next, the processing unit 100 starts acquiring of the result of detection by the pulse wave sensor 10 and the body motion sensor 20 (step A3). The pitch computation unit 120 starts a pitch computation process for computing the pitch of the test subject using the result of detection by the body motion sensor 20 (step A5). The pulse-rate measuring unit 110 also starts a pulse-rate-measuring process for measuring the pulse rate of the test subject using the result of detection of the pulse wave signal by the pulse wave sensor 10 and the result of detection by the body motion sensor 20, and records the result of measurement, as the estimated pulse rate 760, in the memory unit 700 (step A7).

Then, the processing unit 100 performs the pitch-stability-judging process according to the pitch-stability-judging program 720 recorded in the memory unit 700 (step A9).

FIG. 8 is a flow chart showing the flow of the pitch-stability-judging process. First, the stability judging unit 130 judges whether or not it has been possible for the pitch computation unit 120 to compute the pitch (step B1). In an instance in which it is judged that it has been possible to compute the pitch ("Yes" at step B1), the stability judging unit 130 judges whether or not a predetermined stable-pitch condition is satisfied (step B3). The stable-pitch condition can be defined as, e.g., the absolute value of a difference between the pitch that was computed in the current iteration and the pitch that was computed in the previous iteration being less than a predetermined threshold value (or equal to or less than a threshold value). The stable-pitch condition is a condition for judging whether or not the exercise intensity satisfies a predetermined uniform-intensity condition.

In an instance in which it was judged in step B3 that the stable-pitch condition is not satisfied ("No" in step B3), the stability judging unit 130 resets the pitch stability 765 of the memory unit 700 to zero (step B5). The stability judging unit 130 then discontinues the pitch-stability-judging process.

In an instance in which it was judged in step B3 that the stable-pitch condition is satisfied ("Yes" in step B3), the stability judging unit 130 judges whether or not the pitch stability 765 is less than a maximum pitch stability defined in advance (step B7). The maximum pitch stability is the largest value the pitch stability 765 can represent, and can be defined as, e.g., "maximum pitch stability = 5".

In an instance in which it is judged that the pitch stability 765 is less than the maximum pitch stability ("Yes" in step B7), the stability judging unit 130 increments the pitch stability 765 of the memory unit 700 by 1 (step B9). The stability judging unit 130 then discontinues the pitch-stability-judging process.

In an instance in which it is judged in step B1 that it has not been possible to compute the pitch ("No" in step B1), or in an instance in which it is judged in step B7 that the pitch stability 765 is equal to or greater than the maximum pitch stability ("No" in step B7), the stability judging unit 130 then discontinues the pitch-stability-judging process.

Returning to the main process shown in FIG. 7, after the pitch-stability-judging process has been performed, the processing unit 100 performs the pulse-stability-judging process according to the pulse-stability-judging program 730 stored in the memory unit 700 (step A11).

FIG. 9 is a flow chart showing the flow of the pulse-stability-judging process. When the stability judging unit 130 judges that the pulse rate has been measured by the pulse-rate measuring unit 110 ("Yes" in step C1), the stability judging unit 130 judges whether or not a predetermined stable-pulse condition is satisfied (step C3). The stable-pulse condition can be defined as, e.g., the absolute value of a difference between the pulse rate that was measured in the current iteration and the pulse rate that was measured in the previous iteration being less than a predetermined threshold value (or equal to or less than a threshold value). The stable-pulse condition is a condition for judging whether or not the pulse rate satisfies a predetermined approximation condition.

In an instance in which it is judged in step C3 that the stable-pulse condition is not satisfied ("No" in step C), the stability judging unit 130 resets the pulse stability 770 of the memory unit 700 to zero (step C5). Then, the stability judging unit 130 discontinues the pulse-stability-judging process.

In an instance in which it is judged, in step C3, that the stable-pulse condition is satisfied ("Yes" in step C3), the stability judging unit 130 judges whether or not the pulse stability 770 is less than a maximum pulse stability defined in advance (step C7). The maximum pulse stability is the largest value that the pulse stability 770 can represent, and can be defined as, e.g., "maximum pulse stability = 5".

In an instance in which it is judged that the pulse stability 770 is less than the maximum pulse stability ("Yes" in step C7), the stability judging unit 130 increments the pulse stability 770 of the memory unit 700 by 1 (step C9). Then, the stability judging unit 130 discontinues the pulse-stability-judging process.

In an instance in which it is judged in step C1 that the pulse rate could not be measured ("No" in step C1), or in an instance in which it is judged in step C7 that the pulse stability 770 is equal to or greater than the maximum pulse stability ("No" in step C7), the stability judging unit 130 discontinues the pulse-stability-judging process.

Returning to the main process shown in FIG. 7, after the pulse-stability-judging process has been performed, the stable-state judging unit 140 performs a stable-state judging process (step A13). Specifically, the stable-state judging unit 140 judges whether or not a stable-state condition that has been defined in advance is satisfied. The stable-state condition is defined as, e.g., "pitch stability = maximum pitch stability and pulse stability = maximum pulse stability." In other words, the state of the test subject is judged to be in a stable state when the pitch stability 765 and the pulse stability 770 stored in the memory unit 700 are at the largest values that each of these parameters is capable of representing. In an instance in which the stable-state condition is not satisfied, the state of the test subject is judged to be in an unstable state. It shall be apparent that the stable-state condition can also be another condition, such as the pitch stability being equal to or greater than a predetermined value and the pulse stability being equal to or greater than a predetermined value.

Next, the processing unit 100 judges whether or not a pulse rate unmeasurability condition is satisfied (step A15). The pulse rate unmeasurability condition is a condition in which it is impossible for the pulse rate to be measured. The processing unit 100 judges, on the basis of the pulse waveform outputted from the pulse-waveform shaping circuit unit 40 or the signal strength of the pulse wave signal detected by the pulse wave sensor 10, whether or not it has become impossible for the pulse rate to be measured.

In an instance in which it is judged that the pulse rate unmeasurability condition is not satisfied ("No" in step A15), the processing unit 100 judges the last pulse rate to be measured by the pulse-rate measuring unit 110 to be the minimum pulse rate, and renews the minimum pulse rate 780 in the memory unit 700 (step A17). Then, the display controller 160 performs a control for causing the display unit 300 to display the latest estimated pulse rate 760 (step A19), and subsequently proceeds to step A25.

In an instance in which it is judged that the pulse rate unmeasurability condition is satisfied ("Yes" in step A15), the processing unit 100 performs the pulse rate estimation process according to the pulse rate estimation program 750 stored in the memory unit 700 (step A21).

FIG. 10 is a flow chart showing the flow of the pulse rate estimation process. First, the pulse rate estimation unit 150 judges the current state of the test subject on the basis of the result of the judgement in step A13 (step D1). This step D1 is a step for determining whether or not to estimate the pulse rate in a transient state.

In an instance in which it is judged that the state of the test subject corresponds to a stable state, the test subject is thought to be in a rest state or in a steady exercise state. Therefore, no estimation of the pulse rate is performed using a pulse rate estimation model. In contrast, in an instance in which it is judged that the test subject is in an unstable state, the test subject is thought to be in a transient state such as at the start of exercise or end of exercise. Therefore, estimation of the pulse rate is performed using the pulse rate estimation model.

Specifically, in an instance in which the test subject is in a stable state ("stable state" in step D1), the pulse rate estimation unit 150 maintains the pulse rate from the previous iteration as the estimated pulse rate 790 (step D17). This corresponds to using, as the estimated pulse rate, the pulse rate corresponding to an instance in which the test subject is judged to be in a stable state, within a period where, after the stable-state judging unit 140 judges that the test subject in the stable state (step A13), a measurement cannot be performed by the pulse-rate measuring unit 110 ("Yes" in step A15) and the exercise intensity (pitch) corresponding to an instance in which the test subject has been judged to be in the stable state and the exercise intensity (pitch) computed by the pitch computation unit 120 satisfy a predetermined uniform-intensity condition ("Yes" in step B3).

In an instance in which the test subject is in an unstable state ("unstable state" in step D1), the pulse rate estimation unit 150 uses the pitch of the test subject to obtain the pulse rate according to Equation 1 (step D3). Then, the obtained pulse rate is set as the target pulse rate 785 (step D5).

The pulse rate estimation unit 150 judges whether or not the target pulse rate 785 set in step D5 is lower than the minimum pulse rate 780 of the memory unit 700 (step D7). In an instance in which it is judged that the target pulse rate 785 is lower ("Yes" in step D7), the pulse rate estimation unit 150 renews the target pulse rate 785 with the minimum pulse rate 780 (step D9). In an instance in which it is judged that the target pulse rate 785 is equal to or greater than the minimum pulse rate 780 ("No" in step D7), the flow proceeds to step D11 with no action being taken.

Next, the pulse rate estimation unit 150 judges whether or not the reference pulse rate 775 is lower than the target pulse rate 785 (step D11). This step is for judging whether the pulse rate is increasing or decreasing.

If the reference pulse rate 775 is lower than the target pulse rate 785, it can be determined that a state is present in which the test subject has started exercise and the pulse rate is increasing. This state is a transient state in which the pulse rate is increasing rapidly following a rapid rise in the exercise intensity (pitch). In contrast, if the reference pulse rate 775 is equal to or greater than the target pulse rate 785, it can be determined that a state is present in which the test subject has ended the exercise and the pulse rate is decreasing. This state is a transient state in which the pulse rate is decreasing gradually following a rapid decline in the exercise intensity (pitch).

In an instance in which the reference pulse rate 775 is lower than the target pulse rate 785 ("Yes" in step D11), the pulse rate estimation unit 150 uses the start-of-exercise model shown in FIG. 7 to estimate the pulse rate, and renews the estimated pulse rate 790 of the memory unit 700 (step D 13). Then, the pulse rate estimation unit 150 discontinues the pulse rate estimation process.

In an instance in which the reference pulse rate 775 is equal to or greater than the target pulse rate 785 ("No" in step D11), the pulse rate estimation unit 150 estimates the pulse rate on the basis of the end-of-exercise model shown in FIG. 8, and renews the estimated pulse rate 790 of the memory unit 700 (step D 15). Then, the pulse rate estimation unit 150 discontinues the pulse rate estimation process.

Returning to the main process shown in FIG. 7, after the pulse rate estimation process has been performed in step A21, the display controller 160 performs a control for causing the display unit 300 to display the estimated pulse rate 790 in the memory unit 700 (step A23).

After step A19 or step A23, the processing unit 100 judges whether or not to discontinue the process (step A25). For example, it is judged whether or not the test subject has performed, through the operation unit 200, a command operation to discontinue measurement of the pulse rate.

In an instance in which it is judged that the process is not to be discontinued yet ("No" in step A25), the processing unit 100 returns to step A9. In an instance in which it is judged that the process is to be discontinued ("Yes" in step A25), the processing unit 100 discontinues the main process.

## 6. Effect

In the pulse rate monitor 1, the pulse-rate measuring unit 110 measures the pulse rate using the result of detection by the pulse wave sensor 10, and the pitch computation unit 120 computes the pitch of the test subject using the result of detection by the body motion sensor 20. The pulse rate estimation unit 150 uses the pitch that has been computed by the pitch computation unit 120 to estimate the estimated pulse rate. The display controller 160 performs a control so as to cause the display unit 300 to display a pulse rate measured by the pulse-rate measuring unit 110 in an instance in which measurement by the pulse-rate measuring unit 110 is possible, and to cause the display unit 300 to display an estimated pulse rate estimated by the pulse rate estimation unit 150 in an instance in which measurement by the pulse-rate measuring unit 110 is not possible.

The pitch is an exercise intensity in which the vigorousness of the exercise being performed by the test subject is represented by a physical measure (physical exercise intensity), and correlates with the pulse rate of the test subject. Therefore, by using the pitch calculated by the pitch computation unit 120, it is possible to estimate the pulse rate with a high degree of accuracy, even in an instance in which measurement of the pulse rate is difficult.

The pulse rate estimation unit 150 obtains the target pulse rate, which is a provisional pulse rate, using the pitch calculated by the pitch computation unit 120. Then, the estimated pulse rate is obtained so as to progressively approach, in accordance with the passage of time, the target pulse rate from the last pulse rate to be measured by the pulse-rate measuring unit 110.

In a situation in which the pulse rate is increasing, such as at the start of exercise, the pulse rate tends to rise relatively quickly and then gradually stabilize. Therefore, in an instance in which the reference pulse rate is lower than the target pulse rate, the estimated pulse rate is obtained so as to change (increase) logarithmically in accordance with the passage of time, whereby the pulse rate can be estimated so as to follow the actual time change in the pulse rate.

In a situation in which the pulse rate is falling, such as at the end of exercise, the pulse rate tends to initially drop relatively insignificantly, rapidly fall after a certain time has elapsed, and then slowly converge. Therefore, in an instance in which the reference pulse rate is higher than the target pulse rate, the estimated pulse rate is obtained so as to change (decrease) sigmoidally in accordance with the passage of time, whereby the pulse rate can be estimated so as to follow the actual time change in the pulse rate.

## 7. Modification examples

It shall be apparent that examples to which the present invention can be applied is not limited to the above-mentioned example, and that modifications can be made as appropriate without departing from the scope of the present invention. Examples of modification will now be described.

### 7-1 Biological information processing device

In the above-mentioned embodiment, a description was given using a wristwatch-type pulse rate monitor as an example of a biological information processing device; however, a biological information processing device to which the present invention can be applied is not limited to that described. For example, the present invention can also be applied to a finger-worn pulse rate monitor, which is worn on the finger when the pulse rate is measured. The method for detecting the pulse wave signal is not limited to a detection method in which light is used; a detection method in which ultrasound is used, or a detection method in which infrared light is used, is also possible. 7-2 **Body-motion-detector**

In the above-mentioned embodiment, it was described that the body motion sensor, which is the body-motion-detector, is configured so as to have at least an acceleration sensor. However, the body motion sensor may also be configured so as to have another sensor instead of an acceleration sensor. For example, the body motion sensor may be configured so as to have a velocity sensor, wherein the travel velocity of the test subject is detected as the body motion.

In this instance, the pitch $p$ of the test subject is computed according to, e.g., Equation (5) using a travel velocity $v$ detected by the body motion sensor and the height h of the test subject. Then, as described in the "principle" section, the pitch $p$ is used to estimate the pulse rate. It is also possible to calculate the acceleration of the test subject using, as a time derivative, the travel velocity $v$ detected by the body motion sensor, and compute the pitch $p$ from the calculated acceleration, and estimate the pulse rate.

The body motion sensor may also be configured so as to have both an acceleration sensor and a velocity sensor, where the acceleration of the test subject detected by the acceleration sensor and the velocity of the test subject detected by the velocity sensor are used alongside to estimate the pulse rate in a similar manner to that described above.

**7-3 Minimum pulse rate**

In the above-mentioned embodiment, a description was given in which a minimum value (minimum pulse rate) of the provisional pulse rate (target pulse rate) is the lowest pulse rate measured by the pulse-rate measuring unit 110. However, a value that can be set as the minimum pulse rate is not limited to this example.

For example, the lowest value from among the pulse rates measured within a predetermined time (e.g., within 2 minutes) after pulse rate measurement has started can be used as the minimum pulse rate. Alternatively, a value that is higher than the rest pulse rate by a predetermined ratio (e.g., 20%) can be used as the minimum pulse rate.

Also, the test subject can be expected to warm up when starting exercise. In such an instance, there is a possibility that the pulse rate has already increased to at least a certain extent by the time the measurement has started. Therefore, using the lowest pulse rate measured by the pulse-rate measuring unit 110 as the minimum pulse rate may result in a situation in which a pulse rate that is unnecessarily high is used as the target pulse rate. In order to avoid this problem, it is effective to perform an adjustment such as in an instance in which the lowest pulse rate at the start of measurement exceeds a threshold pulse rate defined as a pulse rate that is higher than the rest pulse rate by a predetermined ratio (e.g., 30%), the target pulse rate is set to the threshold pulse rate.

**7-4 Pulse rate estimation model**

The pulse rate estimation model described in the above-mentioned embodiment is only an example. In other words, it shall be apparent that a pulse rate estimation model can be modelled using a function other than the function applied to the model in the above-mentioned embodiment, as long as the function simulates the time change in the pulse rate shown in FIG. 5.

**Claims**

1. A biological information processing device, comprising:

   a measuring unit that measures the pulse rate of a test subject;
   a detector that detects a body motion of the test subject;
   a computation unit that compute an intensity of exercise performed by the test subject using a result of detection by the detector;
   a first estimation unit that estimates an estimated pulse rateusing the exercise intensity computed by the computation unit; and
   a controller that controls so that the pulse rate from a result of the measurement is displayed in an instance in which measurement by the measuring unit is possible, and the estimated pulse rate estimated by the first estimation unit is displayed in an instance in which measurement by the measuring unit is not possible.

2. The biological information processing device according to claim 1, wherein
   the first estimation unit has a second estimation unit that obtains a provisional pulse rate using the exercise intensity computed by the computation unit; and
   obtains the estimated pulse rate so as to progressively approach, in accordance with the passage of time, the provisional pulse rate from a last pulse rate to be measured by the measuring unit.

3. The biological information processing device according to claim 2, wherein
   the second estimation unit obtains, in an instance in which the last pulse rate to be measured by the measuring unit is lower than the provisional pulse rate, obtains the estimated pulse rate so that the pulse rate changes logarithmically in accordance with the passage of time.

**4.** The biological information processing device according to claim 2, wherein
the second estimation unit obtains, in an instance in which the last pulse rate to be measured by the measuring unit is higher than the provisional pulse rate, obtains the estimated pulse rate so that the pulse rate changes sigmoidally in accordance with the passage of time.

**5.** The biological information processing device according to any of claims 2-4, wherein
in the second estimation unit, a minimum value of the provisional pulse rate is a lowest pulse rate measured by the measuring unit.

**6.** The biological information processing device according to any of claims 1-5, wherein
the detector is configured so as to have at least one of an acceleration sensor and a velocity sensor.

**7.** The biological information processing device according to any of claims 1-6, wherein
the detector is configured so as to have at least an acceleration sensor, and
the computation unit calculates a pitch of the test subject from an acceleration detected by the acceleration sensor, and thereby computes the intensity of exercise performed by the test subject.

**8.** The biological information processing device according to any of claims 1-7, further comprising
a judging unit that judges, on the basis of the exercise intensity computed by the computation unit and the pulse rate measured by the measuring unit, whether or not the test subject is in a stable state; wherein
the first estimation unit has a third estimation unit in which the pulse rate corresponding to an instance in which the test subject is judged to be in the stable state is used as the estimated pulse rate within a period where, after the judging unit judges that the test subject in the stable state, a measurement cannot be performed by the measuring unit, and the exercise intensity corresponding to an instance in which the test subject has been judged to be in the stable state and the exercise intensity computed by the computation unit satisfy a predetermined condition of equivalent intensity.

Fig. 1

Fig. 2A

Fig. 2B

**Fig. 3**

Fig. 4

Fig. 5

ESTIMATED PULSE RATE HR

START-OF-EXERCISE MODEL

TARGET PULSE RATE
$HR_{tar}$

REFERENCE PULSE RATE
$HR(t_c)$

REFERENCE TIME
$t_c$

ELAPSED TIME
$t$

Fig. 6A

ESTIMATED PULSE RATE HR

END-OF-EXERCISE MODEL

REFERENCE PULSE RATE
$HR(t_c)$

TARGET PULSE RATE
$HR_{tar}$

REFERENCE TIME
$t_c$

ELAPSED TIME
$t$

$HR_{tar} = HR_{tar}$ (In an instance in which $HR_{tar} \geq HR_{min}$)
$HR_{tar} = HR_{min}$ (In an instance in which $HR_{tar} < HR_{min}$)
Where $HR_{min}$ = MINIMUM PULSE RATE

Fig. 6B

MAIN PROCESS

INITIALIZATION SETTING —A1

START ACQUIRING RESULT OF DETECTION BY PULSE WAVE SENSOR AND BODY MOTION SENSOR —A3

START PITCH COMPUTATION PROCESS —A5

START PULSE-RATE-MEASURING PROCESS —A7

PITCH-STABILITY-JUDGING PROCESS —A9

PULSE-STABILITY-JUDGING PROCESS —A11

STABLE-STATE JUDGING PROCESS —A13

IS PULSE RATE UNMEASURABILITY CONDITION SATISFIED? —A15

No → JUDGE/RENEW MINIMUM PULSE RATE —A17

CONTROL FOR DISPLAY OF MEASURED PULSE RATE —A19

Yes → PULSE RATE ESTIMATION PROCESS —A21

CONTROL FOR DISPLAY OF ESTIMATED PULSE RATE —A23

DISCONTINUE PROCESS? —A25

No

Yes

END

**Fig. 7**

Fig. 8

Fig. 9

Fig. 10

EP 2 520 222 A1

Europäisches
Patentamt
European
Patent Office
Office européen
des brevets

# EUROPEAN SEARCH REPORT

Application Number

EP 12 16 6550

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | US 2010/179389 A1 (MORONEY III RICHARD M [US] ET AL) 15 July 2010 (2010-07-15)<br>* figures 10,11 *<br>* paragraph [0046] *<br>* paragraph [0045] * | 1-7<br><br>8 | INV.<br>A61B5/0205<br>A61B5/024<br>A61B5/11 |
| Y<br><br>A | WO 2007/053146 A1 (UNIV GEORGIA STATE RES FOUND [US]; MARTIN JAMES S [US]; LARSEN JAMES W) 10 May 2007 (2007-05-10)<br>* page 20, line 24 - page 21, line 14 * | 8<br><br>1 | |
| A | US 4 312 358 A (BARNEY GEORGE M) 26 January 1982 (1982-01-26)<br>* column 8, line 17 - line 45 * | 1 | |
| A | US 6 241 684 B1 (AMANO KAZUHIKO [JP] ET AL) 5 June 2001 (2001-06-05)<br>* column 14, line 5 - column 15, line 18 *<br>* column 11, line 14 - line 61 * | 1 | |
| A | EP 1 908 402 A1 (ETA SA MFT HORLOGERE SUISSE [CH]) 9 April 2008 (2008-04-09)<br>* paragraph [0027] - paragraph [0028] *<br>* figure 3 *<br>* paragraph [0031] * | 1-5 | TECHNICAL FIELDS SEARCHED (IPC)<br><br>A61B |
| A | EP 2 070 473 A1 (POLAR ELECTRO OY [FI]) 17 June 2009 (2009-06-17)<br>* figure 4 * | 2-5 | |
| A | US 2007/249949 A1 (HADLEY DAVID M [US]) 25 October 2007 (2007-10-25)<br>* figures 4,5 * | 2-5 | |

The present search report has been drawn up for all claims

| Place of search | Date of completion of the search | Examiner |
|---|---|---|
| The Hague | 2 August 2012 | De la Hera, Germán |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

26

**ANNEX TO THE EUROPEAN SEARCH REPORT**
**ON EUROPEAN PATENT APPLICATION NO.**                    EP 12 16 6550

This annex lists the patent family members relating to the patent documents cited in the above-mentioned European search report.
The members are as contained in the European Patent Office EDP file on
The European Patent Office is in no way liable for these particulars which are merely given for the purpose of information.

02-08-2012

| Patent document cited in search report | | Publication date | Patent family member(s) | | Publication date |
|---|---|---|---|---|---|
| US 2010179389 | A1 | 15-07-2010 | CN | 101400296 A | 01-04-2009 |
| | | | EP | 1991114 A2 | 19-11-2008 |
| | | | JP | 2009528141 A | 06-08-2009 |
| | | | US | 2010179389 A1 | 15-07-2010 |
| | | | WO | 2007100959 A2 | 07-09-2007 |
| WO 2007053146 | A1 | 10-05-2007 | NONE | | |
| US 4312358 | A | 26-01-1982 | JP | 1594650 C | 27-12-1990 |
| | | | JP | 2017170 B | 19-04-1990 |
| | | | JP | 56085326 A | 11-07-1981 |
| | | | US | 4312358 A | 26-01-1982 |
| US 6241684 | B1 | 05-06-2001 | CN | 1194574 A | 30-09-1998 |
| | | | CN | 1545979 A | 17-11-2004 |
| | | | CN | 1589734 A | 09-03-2005 |
| | | | DE | 69725095 D1 | 30-10-2003 |
| | | | DE | 69725095 T2 | 01-04-2004 |
| | | | EP | 0842635 A1 | 20-05-1998 |
| | | | EP | 1338241 A1 | 27-08-2003 |
| | | | JP | 3608204 B2 | 05-01-2005 |
| | | | US | 6241684 B1 | 05-06-2001 |
| | | | WO | 9737588 A1 | 16-10-1997 |
| EP 1908402 | A1 | 09-04-2008 | EP | 1908402 A1 | 09-04-2008 |
| | | | TW | 200833296 A | 16-08-2008 |
| | | | WO | 2008040736 A1 | 10-04-2008 |
| EP 2070473 | A1 | 17-06-2009 | AT | 536132 T | 15-12-2011 |
| | | | EP | 2070473 A1 | 17-06-2009 |
| | | | US | 2009156944 A1 | 18-06-2009 |
| US 2007249949 | A1 | 25-10-2007 | US | 2007249949 A1 | 25-10-2007 |
| | | | WO | 2007124271 A2 | 01-11-2007 |

EPO FORM P0459

For more details about this annex : see Official Journal of the European Patent Office, No. 12/82

**EP 2 520 222 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2011103448 A
- JP 2004283228 A
- JP 2009034366 A
- JP 2004081745 A